# EUROPÄISCHE PATENTANMELDUNG

(11) **EP 0 547 472 A1**
(43) Veröffentlichungstag der Anmeldung: **23.06.1993**
(21) Anmeldenummer: 92120879.9
(22) Anmeldetag: 08.12.1992
(51) Int. Cl.: G01N 33/00, D06F 43/00

(54) **Chlorkohlennwasserstoff-Gassensor mit katalytischer Abspaltung**

(30) Priorität: 18.12.1991 EP 91810987
(71) Anmelder: Endress + Hauser Conducta Gesellschaft für Mess- und Regeltechnik mbH + Co., D-70839 Gerlingen (DE)
(72) Erfinder: Bytyn, Winfried, Dr., W-4630 Bochum 1 (DE); Dobos, Karoly, Dr., W-4600 Dortmund 30 (DE)
(74) Vertreter: Morstadt, Volker, Dipl.-Ing. c/o Endress + Hauser Flowtec AG

(57) **Zusammenfassung**

Dieser Chlorkohlenwasserstoff-Gassensor dient zur kontinuierlichen Messung der Chlorkohlenwasserstoff-Konzentration in mit einem Chlorkohlenwasserstoff als Reinigungsmittel arbeitenden Textilreinigungsmaschinen. Er enthält eine Abspalteinheit (1) zur katalytischen Abspaltung von Kohlenmonoxid und/oder Chlor aus dem Chlorkohlenwasserstoff und einen mit der Abspalteinheit (1) her-metisch dicht verbundenen Kohlenmonoxid- und/oder Chlor-Sensor (2), wobei in einer Stirnseite (3) der Abspalteinheit (1) ein Gaseinlaß (4) angeordnet ist, dessen Querschnittsfläche so dimensioniert ist, daß trotz des in der Textilreinigungsmaschine herrschenden Partialdrucks des Chlorkohlenwasserstoffs die Gasdiffusion in der Abspalteinheit (1) nicht unterbunden ist.

## Beschreibung

Die Erfindung betrifft Chlorkohlenwasserstoff-Gassensoren zur kontinuierlichen Messung der Chlorkohlenwasserstoff-Konzentration in Textilreinigungsmaschinen.

In der Zeitschrift "Hard and Soft", 1989, Heft 11/12, Fachteil Mikroperipherik ist ein Chlorkohlenwasserstoff-Gassensor zur kontinuierlichen Messung der chlorkohlenwasserstoff-Konzentration in Arbeitsräumen beschrieben, in denen mit einem Chlorkohlenwasserstoff als Reinigungsmittel arbeitende Textilreinigungsmaschinen aufgestellt sind. Der Chlorkohlenwasserstoff-Gassensor enthält eine Abspalteinheit zur katalytischen Abspaltung von Kohlenmonoxid und/oder Chlor aus dem Chlorkohlenwasserstoff und einen der Abspalteinheit nachgeordneten Kohlenmonoxid- und/oder Chlor-Sensor in einem gemeinsamen, rohrförmigen Gehäuse. Die Raumluft mit den zu messenden Gasanteilen hat durch einen in einer Stirnseite der Abspalteinheit angeordneten großflächigen Gaseinlaß, z.B. in Form von mehreren Bohrungen entsprechend großen Durchmessers, hindurch Zutritt.

In Textilreinigungsmaschinen werden flüssige Chlorkohlenwasserstoffe, wie z.B. Perchlorethylen (C₂Cl₄) oder Trichlorethylen (C₂HCl₃) (nach neuerem Sprachgebrauch: Perchlorethen oder Trichlorethen), als Reinigungsmittel verwendet. Da diese leicht verdampfen und, insb. deren Dämpfe, schon in geringen Konzentrationen giftig sind, ist für die einzelnen Reinigungsmittel jeweils ein maximal zulässiger Arbeitsplatz-Konzentrationswert, der sogenannte MAK-Wert, für die Luft des Raumes festgelegt, in dem die Textilreinigungsmaschine aufgestellt ist. Für Perchlorethen ist in Deutschland der MAK-Wert derzeit auf 50 ppm (= 5x10⁻³ Vol%) festgesetzt. Die Überschreitung des MAK-Wertes muß nach gesetzlicher Vorschrift überwacht werden, wozu z.B. die eingangs erwähnten Gassensoren dienen können.

Da diese Raumluft-Gassensoren jedoch nur die sehr niedrigen Konzentrationen bzw. Partialdrücke im Bereich des MAK-Wertes messen sollen, sind sie so ausgelegt, daß die Gasmoleküle des Reinigungsmittels durch das Gehäuse hindurch ungehindert zur Abspalteinheit gelangen, z.B. diffundieren, können, wobei die Raumlauft das Gehäuse auch allfällig durchströmen kann, vgl. z.B. die Anordnung nach der CA-A 11 29 959, bei der der Gaseinlaß einen Durchmesser von 1/4 Zoll, also 6,35 mm, hat, der gasempfindliche Teil jedoch ein Halbleiterbauelement ist.

Für den direkten Einsatz in der Textilreiniungsmaschine selbst, insb. an einer geeigneten Stelle des Reinigungsmittelkreislaufs, sind diese Gassensoren jedoch unbrauchbar, da der in der Maschine herrschende Gas-Überdruck des Reinigungsmittels oder ein auftretender Unterdruck, der Raumluft in die Maschine einströmen läßt, zur Abspalteinheit gelangen könnten und damit deren Diffusions-Funktion stark beeinträchtigen oder sogar unterbinden würden.

Die in den Ansprüchen definierte Erfindung dient der Lösung dieses Problems.

Die Erfindung besteht somit nach Anspruch 1 in einem Chlorkohlenwasserstoff-Gassensor zur kontinuierlichen Messung der Chlorkohlenwasserstoff-Konzentration in mit einem Chlorkohlenwasserstoff als Reinigungsmittel arbeitenden Textilreinigungsmaschinen, der eine Abspalteinheit zur katalytischen Abspaltung von Kohlenmonoxid und/oder Chlor aus dem Chlorkohlenwasserstoff und einen mit der Abspalteinheit hermetisch dicht verbundenen Kohlenmonoxid- und/oder Chlor-Sensor enthält, wobei in einer Stirnseite ein Gaseinlaß angeordnet ist, dessen Querschnittsfläche so dimensioniert ist, daß trotz des in der Textilreinigungsmaschine herrschenden Partialdrucks des Chlorkohlenwasserstoffs die Gasdiffusion in der Abspalteinheit nicht unterbunden ist.

Die Erfindung wird nun anhand eines Ausführungsbeispiels unter Bezugnahme auf die Figuren der Zeichnung näher erläutert, in der gleiche Teile mit denselben Bezugszeichen versehen sind.
- Fig. 1: zeigt schematisch in perspektivischer Ansicht ein Ausführungsbeispiel eines Gassensors, und
- Fig. 2: zeigt die Draufsicht auf die in Fig.1 nicht sichtbare Unterfläche des Gassensors.

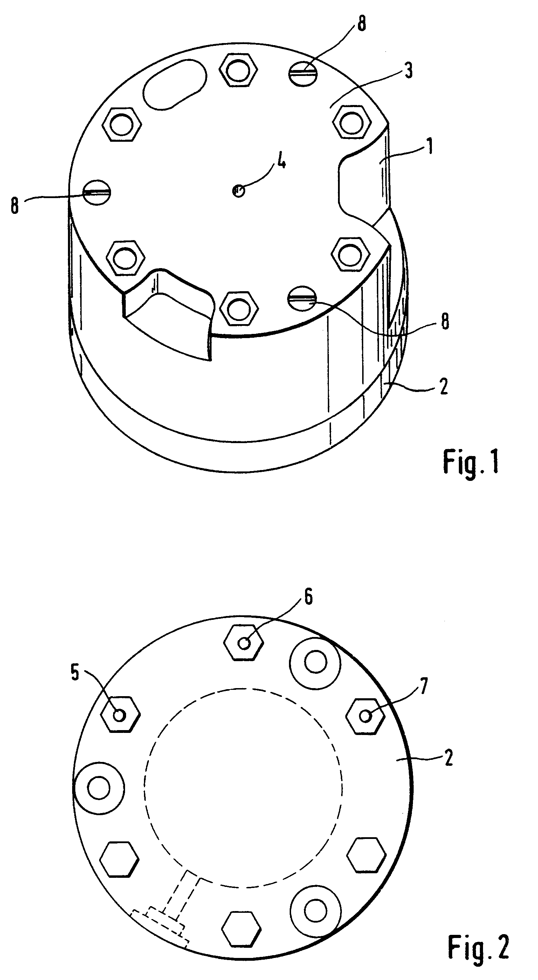

Der in Fig. 1 perspektivisch geteigte Chlorkohlenwasserstoff-Gassensor umfaßt eine Abspalteinheit 1 zur katalytischen Abspaltung von Kohlenmonoxid und/oder Chlor aus dem Chlorkohlenwasserstoff und einen der Abspalteinheit 1 nachgeordneten Kohlenmonoxid- und/oder Chlor-Sensor 2. Dieser Sensor 2 ist, z.B. unter Zwischenlage eines O-Rings als innere Dichtung, mit der Abspalteinheit 1 hermetisch dicht verschraubt ist, so daß ein rohrförmiges dichtes Gehäuse gebildet ist.

Lediglich in einer Stirnseite 3 der Abspalteinheit 1 ist ein Gaseinlaß 4 für die zu messende Innenatmosphäre der Textilreinigungsmaschine angeordnet. Die Querschnittsfläche des Gaseinlasses 4 ist so dimensioniert, daß trotz des in der Textilreinigungsmaschine herrschenden Partialdrucks des Chlorkohlenwasserstoffs die Gasdiffusion in der Abspalteinheit nicht unterbunden ist. Bei ausgeführten Chlorkohlenwasserstoff-Gassensoren lag dieser Durchmesser zwischen 0,2 mm und 1 mm, bevorzugt bei 0,8 mm; der Durchmesser ist also praktisch eine Größenordnung kleiner als der der Anordnung nach der oben genannten CA-A 11 29 959.

Die Fig. 2 zeigt die Unteransicht des Chlorkohlenwasserstoff-Gassensors von Fig. 1; es ist die Stirnfläche des Kohlenmonoxid- und/oder Chlor-Sensors 2. Hier sind drei Anschlußzapfen 5, 6, 7 zu erkennen, die mit einer Arbeitselektrode bzw. einer Referenzelektrode bzw. Gegenelektrode des Sensors 2 verbunden sind und über die der Chlorkohlenwasserstoff-Gassensor z.B. in einer potentiostatischen Schaltung betrieben werden kann.

Mittels Schrauben 8 kann die hermetisch dichte Verbindung zwischen Abspalteinheit 1 und Sensor 2 gelöst werden. Dadurch kann die Abspalteinheit 1 nach dem Aufbrauch ihrer chemischen Bestandteile erneuert werden.

## Patentansprüche

1. Chlorkohlenwasserstoff-Gassensor zur kontinuierlichen Messung der chlorkohlenwasserstoff-Konzentration in mit einem Chlorkohlenwasserstoff als Reinigungsmittel arbeitenden Textilreinigungsmaschinen, der eine Abspalteinheit (1) zur katalytischen Abspaltung von Kohlenmonoxid und/oder Chlor aus dem Chlorkohlenwasserstoff und einen mit der Abspalteinheit (1) hermetisch dicht verbundenen Kohlenmonoxid- und/oder Chlor-Sensor (2) enthält, wobei in einer Stirnseite (3) der Abspalteinheit (1) ein Gaseinlaß (4) angeordnet ist, dessen Querschnittsfläche so dimensioniert ist, daß trotz des in der Textilreinigungsmaschine herrschenden Partialdrucks des Chlorkohlenwasserstoffs die Gasdiffusion in der Abspalteinheit (1) nicht unterbunden ist.

2. Chlorkohlenwasserstoff-Gassensor nach Anspruch 1 mit einem Durchmesser des Gaseinlasses (4), der zwischen 0,2 mm und 1 mm, vorzugsweise 0,8 mm, beträgt.

3. Chlorkohlenwasserstoff-Gassensor nach Anspruch 1 oder 2 mit einer in oder an der Abspalteinheit angeordneten Heizvorrichtung.
